# EUROPEAN PATENT APPLICATION

(11) **EP 4 775 988 A1**
(43) Date of publication of application: **15.07.2026**
(21) Application number: 24901182.6
(22) Date of filing: 15.11.2024
(51) Int. Cl.: G01N 33/543, G01N 33/536, B01L 3/00

(54) **METHOD OF PERFORMING LIQUID IMMUNOASSAY BY USING IN VITRO DIAGNOSTIC CARTRIDGE HAVING MAGNETIC BEAD WELL, REACTION WELL, WASHING WELL AND MEASUREMENT WELL**

(30) Priority: 06.12.2023 KR 20230175156
(71) Applicant: Boditech Med Inc., Chuncheon-si, Gangwon-do 24398 (KR)
(72) Inventor: HA, Yong Hwang, Chuncheon-si, Gangwon-do 24403 (KR); CHO, Chu Hyun, Chuncheon-si, Gangwon-do 24209 (KR); OH, Young Jin, Chuncheon-si, Gangwon-do 24401 (KR); JUNG, Ji Woon, Chuncheon-si, Gangwon-do 24278 (KR); YEO, Kang In, Yeongcheon-si, Gyeongsangbuk-do 38837 (KR); YOO, Jin, Chuncheon-si, Gangwon-do 24416 (KR)
(74) Representative: Louis Pöhlau Lohrentz
(86) International application number: PCT/KR2024/096584
(87) International publication number: WO 2025/121996

(57) **Abstract**

The present invention relates to a method for performing liquid immunoassay using an in vitro diagnostic cartridge having a magnetic bead well, a reaction well, a washing well, and a measurement well. The liquid immunoassay method of the present invention comprises: a step of bringing a magnet close to a pipette tip to collect magnetic beads filled in the magnetic bead well and transferring the collected magnetic beads to the reaction well; a step of introducing a sample into the reaction well to perform an antigen-antibody reaction; a step of bringing the magnet close to the pipette tip to collect a reaction product in the reaction well onto the pipette tip and transferring the collected reaction product to the washing well for washing; and a step of bringing the magnet close to the pipette tip to transfer the washed reaction product from the washing well to the measurement well for optical analysis. According to the present invention, the collection yield of the magnetic beads can be improved.

## Description

### TECHNICAL FIELD

The present invention relates to a method for performing liquid immunoassay using an in vitro diagnostic cartridge having a magnetic bead well, a reaction well, a washing well, and a measurement well, and more particularly, to a liquid immunoassay method that improves the collection yield of magnetic beads.

### BACKGROUND ART

With advances in medicine, biotechnology, and various related technologies, tests for detecting a variety of molecular indicators-such as blood cells, genes, proteins, antigens, and pathogens-from predetermined biological samples such as urine and blood have become widely practiced. The testing process generally involves collecting a sample, reacting the collected sample with predetermined reagents appropriate for the target indicator, and then analyzing and observing the resulting changes. Through this process, qualitative and/or quantitative analysis of various molecular indicators contained in the sample is possible, thereby obtaining information regarding disease diagnosis, disease progression, or prognosis.

One technique widely used in such testing processes is the immunoreaction technique, also referred to as Enzyme ImmunoAssay (EIA), which is based on the specific binding between antigens and antibodies. Depending on the type of substrate used for detecting the analyte, this includes colorimetric measurement methods (chromogenic or colorimetric) that measure color changes by absorbance, chemiluminescence methods, and fluorescence-based methods. Additionally, depending on the analytical approach, there are sandwich-type immunoreactions and competitive immunoreactions, the former being also known as Enzyme Linked Immunosorbent Assay (ELISA).

Regardless of the method used in such analyses, removal of non-specific reactants is desirable for high-sensitivity detection with high specificity. That is, after reaction of reagents with a sample during a test, purification or separation of the reaction products is necessary for accurate detection of the reaction products.

The most effective method for removing non-specific reactants is physical washing or purification. Among these, it is preferable to remove non-specific reactants using a washing method utilizing magnetic particles. Such a washing method using magnetic particles requires a separate apparatus for separation and washing, and can remove non-specific reactants by repeatedly performing dispersion and collection of magnetic particles. However, the method of repeatedly dispersing and collecting magnetic particles requires a lengthy washing process and has the disadvantage of losing many magnetic beads. Therefore, further development of test apparatus and methods for effective removal of non-specific reactants within a short time without losing magnetic beads remains necessary.

Meanwhile, disposable cartridges pre-filled with reagents are widely used in Point of Care (POC) in vitro diagnostic equipment. Such disposable cartridge frames include a frame made of plastic, combined with wells (e.g., reagent wells, reaction wells, washing wells, measurement wells, and sample wells) necessary for filling reagents and performing various reactions and purifications, as required.

In POC analysis using cartridges, liquid-phase immunoassay technology utilizing magnetic beads (MB) for separation and purification of samples is becoming increasingly important. Using an antigen/antibody pair or an antibody/antibody pair, one type of antibody or antigen is conjugated to magnetic beads, which are then used to form antigen-antibody complexes. Permanent magnets are subsequently used to separate and purify the target analyte with high sensitivity for quantitative or qualitative analysis. In liquid immunoassay technology, magnetic beads are a critical material for high-purity purification of target analytes; in particular, the technology for increasing the effective collection yield of magnetic beads is very important as it is directly related to the overall sensitivity and specificity of the analysis.

### SUMMARY OF THE INVENTION

### Technical Problem

One object of the present invention is to provide a liquid immunoassay method that increases the effective collection yield of magnetic beads.

Another object of the present invention is to provide a liquid immunoassay method with improved analytical sensitivity and specificity.

### Technical Solution

To achieve the foregoing objects, the present invention provides a method for performing liquid immunoassay using an in vitro diagnostic cartridge having a magnetic bead well, a reaction well, a washing well, and a measurement well, the method comprising: a step of bringing a magnet close to a pipette tip to collect magnetic beads filled in the magnetic bead well and transferring the collected magnetic beads to the reaction well; a step of introducing a sample into the reaction well to perform an antigen-antibody reaction; a step of bringing the magnet close to the pipette tip to collect a reaction product in the reaction well onto the pipette tip and transferring the collected reaction product to the washing well for washing; and a step of bringing the magnet close to the pipette tip to transfer the washed reaction product from the washing well to the measurement well for optical analysis.

Preferably, the pipette tip is mounted on the in vitro diagnostic cartridge.

Preferably, the reaction product is aspirated into the pipette tip, and the magnet is brought close to the pipette tip from the outside of the pipette tip so that the reaction product is collected onto the pipette tip.

Preferably, the magnetic bead well is filled with a greater amount of magnetic beads than is required for the optical analysis.

### Technical Effects

The present invention having the foregoing configuration provides a liquid immunoassay method having a high effective collection yield of magnetic beads. The present invention also provides a liquid immunoassay method with high analytical sensitivity and specificity.

### BRIEF DESCRIPTION OF DRAWINGS

FIG. 1 illustrates the process of a sandwich immunoreaction using magnetic beads employed in an apparatus according to one embodiment of the present invention.
FIG. 2 illustrates the process of a competitive immunoreaction using magnetic beads employed in an apparatus according to one embodiment of the present invention.
FIG. 3 is a configuration diagram of an in vitro diagnostic cartridge to which one embodiment of the present invention may be applied.
FIG. 4 is a flowchart of a liquid immunoassay method according to one embodiment of the present invention.
FIG. 5 illustrates a magnetic bead collection method according to one embodiment of the present invention.
FIG. 6 illustrates a case in which the liquid immunoassay method according to one embodiment of the present invention is applied to the cartridge shown in FIG. 3.

### DETAILED DESCRIPTION OF EMBODIMENTS

To fully understand the present invention, preferred embodiments of the present invention will now be described with reference to the accompanying drawings. The embodiments of the present invention may be modified in various forms, and the scope of the present invention should not be construed as being limited to the embodiments described in detail below. The present embodiments are provided to more completely describe the present invention to those of ordinary skill in the art. Accordingly, the shapes of elements shown in the drawings may be exaggerated for clarity of description. It should be noted that the same members in each drawing may be denoted by the same reference numerals. Detailed descriptions of well-known functions and constructions that are deemed to unnecessarily obscure the gist of the present invention are omitted.

In the following, the terminology used herein and the principles of the chemical reactions used with the apparatus are described first.

As used herein, "detection" refers to, in order to determine the presence or amount of an analyte contained in a sample described below, quantitatively or qualitatively analyzing the analyte after reacting reagents with the sample and purifying the resulting reaction product.

As used herein, "test" is used as a term encompassing detection, analysis, and reading.

"Analyte" refers to a compound subject to analysis in a sample, also referred to as a target or an indicator, and includes, but is not limited to, protein components such as antigens or nucleic acid materials such as genes.

As used herein, "reagent" refers to a substance mixed with a sample for quantitative or qualitative analysis of an analyte contained in the sample. The reagent varies depending on the type of specific analyte and may include, but is not limited to, a reaction buffer, a dilution buffer, a detection buffer, a washing buffer, or predetermined antibodies, enzymes, or substrates that react with various substances in the sample, such as antigens.

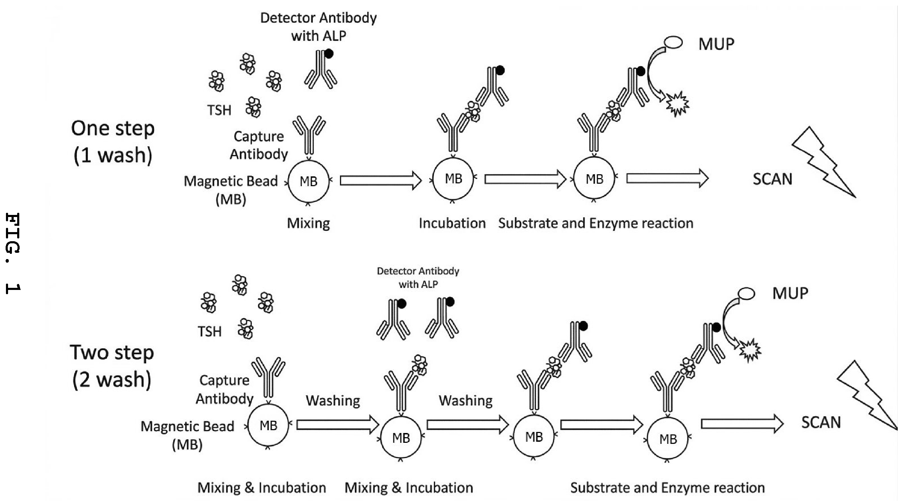

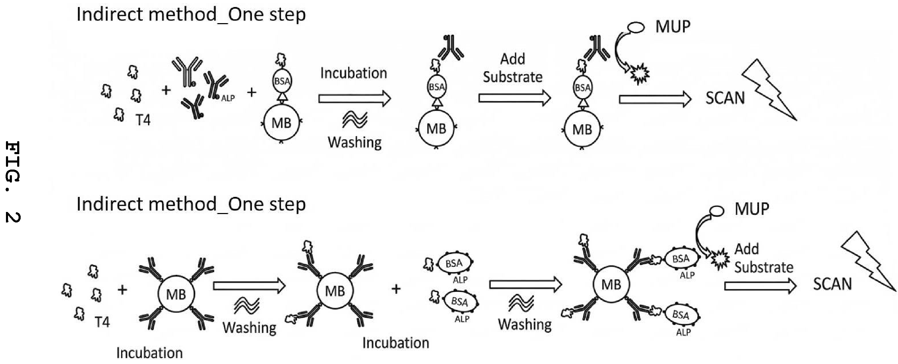

The immunoassay apparatus according to one embodiment of the present invention relates to a method optimized for detecting specific components or analytes contained in biological samples through immunoassay (ELISA)-based reactions founded on the specific binding between antigens and antibodies-for example, reactions such as those shown in FIGS. 1 and 2-and for physical washing using magnetic beads to separate unreacted materials from the reaction products prior to detection of the analyte.

FIGS. 1 and 2 illustrate various types of ELISA analysis processes for analyzing analytes. A sandwich immunoassay refers to an immunoreaction in which a capture antibody and a detector antibody are bound in a sandwich configuration, and an enzyme is chemically conjugated to the detector antibody to induce a quantitative reaction with a substrate. In this case, the capture antibody is chemically or physically bound to magnetic beads, and the detector antibody is used in the form of a conjugate that is bound to an enzyme. The sandwich reaction using such magnetic beads can be broadly categorized into two types depending on the number of washing steps: a one-step assay or a two-step assay. The method of first reacting the sample with the capture antibody, washing, and then reacting with the detector antibody is referred to as a two-step reaction, while the method of simultaneously reacting the capture antibody and the detector antibody without separation is referred to as a one-step reaction.

A competition assay, which together with the sandwich immunoassay is widely used for detecting trace amounts of protein molecules, is also divided into two methods. Depending on whether a competitive protein or antibody is conjugated to the magnetic beads, it is divided into an indirect competitive reaction or a direct competitive reaction; and depending on the number of steps in the immunoreaction, it is divided into a one-step or two-step reaction. For example, FIG. 2 shows one form of an indirect competitive reaction and a direct competitive reaction among competitive reactions.

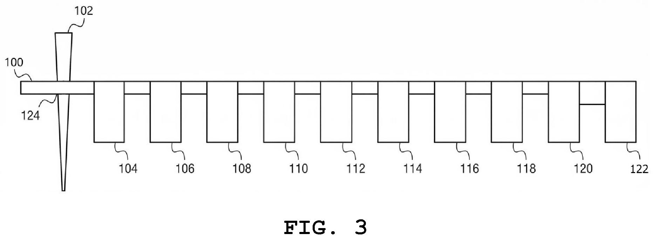

FIG. 3 is a configuration diagram of an in vitro diagnostic cartridge 100 to which one embodiment of the present invention may be applied. As shown, the cartridge 100 comprises a sample well 104, a magnetic bead well 106, reagent wells 108, 116, 118, 120, a reaction well 110, washing wells 112, 114, and a measurement well 122.

A sample is loaded and filled into the sample well 104. Diagnostic reagents necessary for the liquid immunoassay are filled into the reagent wells 108, 116, 118, 120. Magnetic beads conjugated with a reagent are filled into the magnetic bead well 106. It is preferable that the magnetic bead well 106 is filled with a greater amount of magnetic beads than is required for the optical analysis.

In the reaction well 110, magnetic beads conjugated with a reagent, a luminescent material, target components in the sample, and other diagnostic reagents react to form an antigen-antibody complex. Unreacted materials in the sample are removed in the washing wells 112, 114. The measurement well 122 is connected to a signal detection device, and qualitative or quantitative analysis of the target component is performed by measuring the luminescence in the measurement well 122. The pipette tip 102 may be mounted in a pipette tip well 124 formed in the cartridge 100.

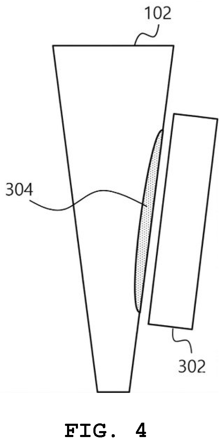

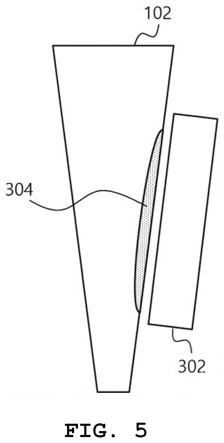

FIG. 4 is a flowchart of a liquid immunoassay method according to one embodiment of the present invention, and FIG. 5 illustrates a magnetic bead collection method according to one embodiment of the present invention.

First, as shown in FIG. 5, a permanent magnet 302 is brought close to the pipette tip 102 to collect the magnetic beads 304 filled in the magnetic bead well 106 using the magnetic force of the permanent magnet 302, and the collected magnetic beads are transferred to the reaction well 110 (step S202). That is, the magnetic beads 304 filled in the magnetic bead well 106 are selectively collected by the pipette tip 102 and the permanent magnet 302 and introduced into the reaction well 110. Through this selective collection process, only the magnetic beads 304 in good condition among all the magnetic beads 304 filled in the magnetic bead well 106 are transferred to the reaction well 110. Next, the sample filled in the sample well 104 is introduced into the reaction well 110, and the reagents filled in the reagent wells 108, 116, 118, 120 are introduced into the reaction well 110 to perform the antigen-antibody reaction (step S204).

Next, the permanent magnet 302 is brought close to the pipette tip 102, and the reaction product bound to the magnetic beads in the reaction well 110 is collected onto the pipette tip 102 and transferred to the washing wells 112, 114 for washing (step S206). The reaction product is aspirated into the pipette tip 102, and by bringing the permanent magnet 302 close to the outside of the pipette tip 102, the reaction product bound to the magnetic beads is collected inside the pipette tip 102. Next, the permanent magnet 302 is brought close to the pipette tip 102, and the reaction product washed in the washing wells 112, 114 is transferred to the measurement well 122 for optical analysis (step S208).

If some magnetic beads 304 are lost during the inter-well transfer process, signal loss occurs. The reason for the loss of magnetic beads 304 during the collection and transfer process is that some magnetic beads 304 react weakly to the permanent magnet 302 and remain in the solution without being attached to the permanent magnet 302. The reason that some magnetic beads 304 react weakly to the permanent magnet 302 is that some magnetic beads 304 are located at a relatively large distance from the permanent magnet 302 and are relatively strongly attached to the surface of the cartridge 100, such that they are not detached and are not collected. Furthermore, it is understood that during storage in an aqueous solution for a period of several months or several tens of months, a slow chemical reaction occurs due to reaction with water, oxygen, and constituents of the solution, and the magnetite-the principal material of the magnetic beads 304-transforms into hematite, which does not exhibit magnetism.

Differences in collection yield of the magnetic beads 304 may also occur due to differences between liquid immunoassay devices using the magnetic beads 304. That is, differences in collection yield may arise from differences in the strength of the permanent magnet between devices, differences in the distance between the permanent magnet and the magnetic beads, and similar factors. A common method for increasing the collection yield of magnetic beads 304 is to increase the strength of the permanent magnet (or electromagnet) 302 used for collecting the magnetic beads 304 or to reduce the gap between the permanent magnet 302 and the magnetic beads 304. However, even with such methods, there is a limit to collecting the magnetic beads 304 that have been chemically denatured and whose response to a magnetic field has been significantly reduced.

In order to correct differences between devices and improve the collection yield, the present embodiment introduces a step of selectively collecting the magnetic beads 304 prior to the step of performing the antigen-antibody reaction in the reaction well 110. In the conventional method, the magnetic beads were simply introduced into the reaction well 110 without using a permanent magnet, and the antigen-antibody reaction was then performed. In the present embodiment, a first step of collecting the magnetic beads from the magnetic bead well 106 using the permanent magnet 302 is performed, and the collected magnetic beads are then transferred to the reaction well 110 where the antigen-antibody reaction takes place. The remaining steps, other than the addition of the selective magnetic bead collection step, are identical to the conventional method.

The pipette tip 102 may be cylindrical or conical in shape. The magnetic bead solution accommodated in the magnetic bead well 106 is aspirated into the pipette tip 102, and then a permanent magnet having a high magnetic force is brought close to the outside of the pipette tip 102 to attach the magnetic beads to the inner wall of the pipette tip 102, after which the remaining solution is discharged, thereby collecting the magnetic beads. This process is repeated 1 to 20 times to concentrate the magnetic beads. After discharging the remaining solution, the magnetic beads are attached to the inner wall of the pipette tip 102, and the pipette tip 102 is then moved to another well; with the magnet removed, the aspiration and discharge process is repeated using the solution in the corresponding well to release the magnetic beads into that well. The magnetic beads are collected and transferred in this manner.

By using the method of selectively collecting magnetic beads, differences between diagnostic devices can be reduced. Even if some magnetic beads have been denatured due to long-term storage and their magnetism has weakened, by collecting the magnetic beads in a preceding step and transferring them to the reaction well 110-rather than directly introducing them into the antigen-antibody reaction-only those magnetic beads having magnetism sufficient for the device to transfer are selectively used, thereby reducing loss during the inter-well dispersion and collection process of the magnetic beads.

During the selective collection process, chemically denatured magnetic beads are not collected, so the amount of magnetic beads collected may decrease. However, since the amount of magnetic beads used in the antigen-antibody reaction is normally in excess rather than a stoichiometric amount, even if some magnetic beads are lost during selective collection, the analysis is not affected.

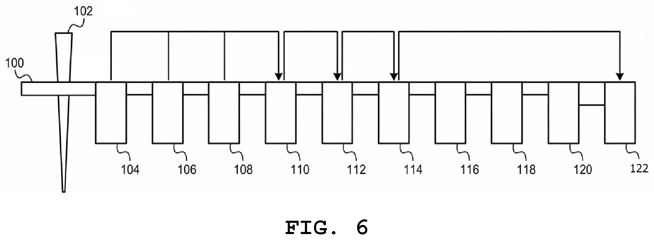

FIG. 6 illustrates a case in which the liquid immunoassay method according to one embodiment of the present invention is applied to the cartridge shown in FIG. 3. In the present embodiment, the magnetic beads are transferred a total of four times: to the reaction well 110, the two washing wells 112, 114, and the measurement well 122.

Table 1 shows a comparative example (Comparative Example 1) in which the magnetic beads in the magnetic bead well 106 were directly introduced into the reaction well 110 without a selective magnetic bead collection process. The absorbance in the reaction well 110 of the first cartridge was 0.3058 and the absorbance in the measurement well 122 was 0.2517. That is, the transfer rate of the magnetic beads in the first cartridge was 82.3%. The transfer rate of the magnetic beads in the second cartridge was 100.7%, and the transfer rate in the third cartridge was 83.8%. Since the transfer rate cannot exceed 100%, the transfer rate of the second cartridge is attributed to signal measurement error.

**[Table 1]**

| | **#1** | **#2** | **#3** |
|---|---|---|---|
| Absorbance in Reaction Well | 0.3058 | 0.3058 | 0.3058 |
| Absorbance in Measurement Well | 0.2517 | 0.3079 | 0.2562 |
| Transfer Rate (%) | **82.3** | **100.7** | **83.8** |

Table 2 shows an example (Example 1) in which the magnetic beads in the magnetic bead well 106 were selectively collected according to the present embodiment and then introduced into the reaction well 110. The absorbance in the reaction well 110 was 0.248433. That is, 85.28% of the magnetic beads were transferred from the magnetic bead well 106 to the reaction well 110 during the selective collection process. In this case, the transfer rate of the magnetic beads was 97.7% in the first cartridge, 89.4% in the second cartridge, and 97.1% in the third cartridge.

**[Table 2]**

| | **#1** | **#2** | **#3** |
|---|---|---|---|
| Absorbance in Reaction Well | 0.248433 | 0.248433 | 0.248433 |
| Absorbance in Measurement Well | 0.2428 | 0.2222 | 0.2413 |
| Transfer Rate (%) | **97.7** | **89.4** | **97.1** |

Table 3 shows the mean transfer rate, standard deviation (SD), and coefficient of variation (CV) for Comparative Example 1 and Example 1. From Table 3, it can be confirmed that the reproducibility in Example 1 is significantly higher than that in Comparative Example 1.

**[Table 3]**

| | **Mean (%)** | **SD** | **CV (%)** |
|---|---|---|---|
| Comparative Example 1 | **88.9** | **10.2** | **11.5** |
| Example 1 | **94.8** | **4.6** | **4.9** |

The embodiments of the present invention described above are merely illustrative, and those of ordinary skill in the art to which the present invention pertains will appreciate that various modifications and equivalent other embodiments are possible therefrom. Therefore, the present invention should be well understood as not being limited only to the forms mentioned in the detailed description above. Accordingly, the true technical scope of protection of the present invention should be determined by the technical spirit of the appended claims. It should also be understood that the present invention includes all modifications, equivalents, and substitutes within the spirit and scope of the present invention as defined by the appended claims.

## Claims

1. A method for performing liquid immunoassay using an in vitro diagnostic cartridge having a magnetic bead well, a reaction well, a washing well, and a measurement well, the method comprising:
bringing a magnet close to a pipette tip to collect magnetic beads filled in the magnetic bead well and transferring the collected magnetic beads to the reaction well;
introducing a sample into the reaction well to perform an antigen-antibody reaction;
bringing the magnet close to the pipette tip to collect a reaction product in the reaction well onto the pipette tip, and transferring the collected reaction product to the washing well for washing; and
bringing the magnet close to the pipette tip to transfer the washed reaction product from the washing well to the measurement well for optical analysis.

2. The liquid immunoassay method of claim 1, wherein the pipette tip is mounted on the in vitro diagnostic cartridge.

3. The liquid immunoassay method of claim 1, wherein the reaction product is aspirated into the pipette tip, and the magnet is brought close to the pipette tip from the outside of the pipette tip so that the reaction product is collected onto the pipette tip.

4. The liquid immunoassay method of claim 1, wherein the magnetic bead well is filled with a greater amount of magnetic beads than is required for the optical analysis.
